(19) Europäisches Patentamt
European Patent Office
Office européen des brevets

(11) EP 2 209 918 B1

(12) **EUROPÄISCHE PATENTSCHRIFT**

(45) Veröffentlichungstag und Bekanntmachung des Hinweises auf die Patenterteilung:
**28.11.2012 Patentblatt 2012/48**

(21) Anmeldenummer: **08852553.0**

(22) Anmeldetag: **29.10.2008**

(51) Int Cl.:
***C12Q 1/68*** *(2006.01)*

(86) Internationale Anmeldenummer:
**PCT/EP2008/064680**

(87) Internationale Veröffentlichungsnummer:
**WO 2009/065711 (28.05.2009 Gazette 2009/22)**

(54) **VERFAHREN ZUR KALIBRIERUNG EINES SENSORELEMENTS**

METHOD FOR CALIBRATING A SENSOR ELEMENT

PROCÉDÉ POUR ÉTALONNER UN ÉLÉMENT CAPTEUR

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **20.11.2007 DE 102007055386**

(43) Veröffentlichungstag der Anmeldung:
**28.07.2010 Patentblatt 2010/30**

(73) Patentinhaber: **Siemens Aktiengesellschaft**
**80333 München (DE)**

(72) Erfinder:
- **FRIEDRICH, Katja**
  **90425 Nürnberg (DE)**
- **GUMBRECHT, Walter**
  **91074 Herzogenaurach (DE)**
- **PAULICKA, Peter**
  **91056 Erlangen (DE)**
- **STANZEL, Manfred**
  **91056 Erlangen (DE)**
- **WEBER, Renee**
  **89129 Langenau-Albeck (DE)**

(56) Entgegenhaltungen:
**EP-A- 1 108 472**
**EP-A- 1 132 485**
**WO-A-02/097413**
**WO-A-2004/106546**
**WO-A-2005/064012**
**WO-A-2007/062666**
**US-A1- 2004 081 974**
**US-A1- 2006 115 851**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 209 918 B1

## Beschreibung

Die Erfindung betrifft ein Verfahren sowie eine Anordnung zur Kalibrierung von Sensorelementen, insbesondere zur Kalibrierung von Sensorelementen auf Mikroarray-Anordnungen.

In der Nukleinsäureanalytik hat die Entwicklung von Mikroarray-basierten Methoden in den letzten Jahren rasante Fortschritte gemacht. Eine Mikroarray-Anordnung ist eine Anordnung von einer Matrix von Sondenmolekülen, welche auf einem Sensor in einzelnen, adressierbaren Positionen angebracht sind, so dass jede Position in der Arrayanordnung ein Sensorelement bildet, mit welchem ein Zielmolekül nachgewiesen werden kann. Bei der Nukleinsäureanalytik werden als Sondenmoleküle üblicherweise Sondenoligonukleotide verwendet, welche z.B. in einer Anordnung in Form eines Chips in einer rasterartigen Matrix auf einem Träger immobilisiert ("gespottet") sind. Durch Hybridisierung mit einer komplementären Ziel-Nukleinsäure kommt es zur Bindung an das Sondenoligonukleotid. Diese Bindung kann dann durch eine Mehrzahl von alternativen Verfahren detektiert werden, so dass aufgrund des Bindungsereignisses die Anwesenheit der Ziel-Nukleinsäure (Z) erfasst und gegebenenfalls auch quantifiziert werden kann. Als Nachweisprinzip kommen dabei optische, elektrochemische, gravimetrische, magnetische und andere geeignete Verfahren zum Einsatz.

Bei optischen Verfahren wird die Ziel-Nukleinsäure bzw. das Hybrid aus Sondenoligonukleotid und Ziel-Nukleinsäure durch ein Label markiert, welches zu einem optisch detektierbaren Signal führt. Dies kann ein Farbstoff, ein Fluorophor, ein Chromophor, ein interkalierender Farbstoff, ein Fluoreszenzfarbstoff oder ähnliches sein. Bei Auftreten eines Bindungsereignisses ist an der adressierbaren Position des jeweiligen Sondenoligonukleotids ein optisch detektierbares Signal erfassbar, z.B. durch eine CCD-Kamera, welche den gesamten Array abbilden kann.

Bei elektrochemischen Nachweisverfahren können die Sondenoligonukleotide auf einem elektrochemischen Sensor immobilisiert werden. Die Ziel-Nukleinsäure bzw. das Hybrid aus Sondenoligonukleotid und Ziel-Nukleinsäure wird durch ein Label markiert, welches an der Position des jeweiligen Sondenoligonukleotids lokal die elektrochemischen Eigenschaften an den Sensorelement verändert, so dass ein elektrisches Signal, z.B. eine Spannung, ein Stromfluss, eine Kapazitätsänderung oder ähnliches gemessen werden kann. Ein entsprechendes Verfahren ist aus DE 101 26 341 bekannt. Hierbei werden Ziel-Nukleinsäuren mit Biotin markiert, nach Hybridisierung der Ziel-Nukleinsäure an das Sondenoligonukleotid wird die gebundene Ziel-Nukleinsäure mit Streptavidin-alkalischer Phosphatase markiert und der alkalischen Phosphatase ein Enzymsubstrat angeboten, welches bei Umsatz durch die Phosphatase ein Produkt ergibt, welches lokal die Leitfähigkeit ändert, so dass an der Elektrode, auf welcher das Sondenoligonukleotid immobilisiert ist, ein lokaler Stromanstieg messbar ist.

Bei gravimetrischen Verfahren wird ein Signal erfasst, welches sich durch die Massenänderung bei Hybridisierung der Ziel-Nukleinsäure an das Sondenoligonukleotid ergibt. Bekannt sind z.B. so genannte FBAR-Verfahren und Kantilever-Verfahren.

Bei einer magnetischen Detektion ist das Sondenoligonukleotid auf einem magnetischen Sensorelement immobilisiert, z.B. einem GMR-Sensor. Die Ziel-Nukleinsäure bzw. das Hybrid aus Sondenoligonukleotid und Ziel-Nukleinsäure kann nun z.B. mit paramagnetischen Partikel markiert werden, z.B. Eisenoxid-Nanopartikel, so dass an der Position des Sondenoligonukleotids bei Bindung der Ziel-Nukleinsäure lokal eine Veränderung der magnetischen Eigenschaften erfassbar ist.

Ein Problem, welches bei sämtlichen Detektionsprinzipien auftaucht, ist die Tatsache, dass zwischen einzelnen Sensorelementen qualitative Unterschiede auftreten können, die gerade bei hoch sensitiven und quantitativen oder semiquantitativen Messverfahren zu unterschiedlich starken Signalstärken führen können. Auch durch andere äußere Einflüsse, z.B. Temperaturschwankungen oder Temperaturgradienten, durch die Fluidik an der Sensoroberfläche bedingte Strömungsschwankungen oder Strömungsgradienten und andere Faktoren, kann es dazu kommen, dass nicht alle Sensorelemente in einer Mikroarray-Anordnung die gleiche Sensitivität aufweisen bzw. bei entsprechend gleicher Konzentration der Ziel-Nukleinsäure (Z) ein Signal mit gleicher Signalstärke liefern. So ist es z.B. bekannt, dass sich Sensorelemente, welche sich am Rand einer Mikroarray-Anordnung befinden, anders verhalten, als Sensorelemente, welche sich in der Mitte einer Mikroarray-Anordnung befinden.

Für den verlässlichen, fehlerfreien Betrieb von auf Mikroarrays basierenden Assays ist also eine verlässliche und fehlerfreie Qualitätskontrolle erforderlich. Diese muss vor allem die wesentlichen Elemente einer Positiv- und Negativkontrolle beinhalten. Hierzu sollte ein jeweiliges Sensorelement im Idealfall mittels einer Zwei-Punktkalibrierung kalibriert werden, d.h. jeder Sensor sollte neben der zu bestimmenden Probe auch noch mit zwei bekannten Proben (-Konzentrationen) beaufschlagt werden und die jeweiligen Messsignale aufgezeichnet werden.

Insbesondere für Mikroarray-Systeme mit einer hohen Anzahl von Sensorelementen bzw. adressierbaren Positionen ist diese Vorgehensweise sehr schwierig zu realisieren, da ein Zwei-Punkt-Kalibriersystem üblicherweise komplexe und kostenintensive Maßnahmen erfordert. In der Technik ist es bekannt, dieses Problem dadurch zu lösen bzw. zu umgehen, indem in der Mikroarray-Anordnung verschiedene Sensorelement-Positionen als Kontroll-Sensorelemente (Kontroll-Spots) vorgesehen werden. Dies hat jedoch den Nachteil, dass dabei angenommen werden muss, dass sich die verschiedenen Sensoren absolut gleich verhalten, und dass die vorgesehenen Kontroll-Spots für alle Sensorelemente repräsentativ sind.

WO 2005/064012 offenbart ein Array-System mit Messfeldern und separaten Kontrollfeldern, wobei auf den Kontrollfeldern andere Fängermoleküle sind als auf den Messfeldern. US 2006/115851 A1 offenbart ein Verfahren zur Anwendung von Kontrollen als Kalibratoren für ein Mikroarray-System worin bestimmte Mengen der Kontrollen unter Hybridisierungsbedingungen dem System zugefügt werden und worin diese unspezifisch an Fängermoleküle binden.
Aufgabe der Erfindung ist die Realisierung einer einfachen, kostengünstigen Zwei-Punkt-Kalibrierung, die jeweils mit ein und demselben Sensorelement durchgeführt werden kann, welches auch als Sensor für eine Ziel-Nukleinsäure verwendet wird. Erfindungsgemäß wird dieses Ziel erreicht durch das Verfahren gemäß Anspruch 1. Vorteilhafte Weiterbildungen der Erfindung sind in den abhängigen Ansprüchen beschrieben.
Die Erfindung betrifft ein Verfahren zur Kalibrierung eines Sensorelements nach Anspruch 1, aufweisend:

a) In Kontakt Bringen des Sensorelements mit einer Mischung aufweisend Kontrollnukleinsäure (K) und Ziel-Nukleinsäure (Z), wobei die Schmelztemperatur der Kontrollnukleinsäure Tm(K) kleiner ist als die Schmelztemperatur der Zielnukleinsäure Tm(Z) wobei Tm (K) die Temperatur ist, unterhalb derer die Kontrollnukleinsäure (K) mit dem immobilisierten Sondenoligonukleotid hybridisiert und wobei Tm (Z) die Temperatur ist, unterhalb derer die Zielnukleinsäure (K) mit dem immobilisierten Sondenoligonukleotid hybridisiert;
b1) Hybridisieren der Mischung an das Sondenoligonukleotid bei einer Temperatur T[p] < Tm(K), und erfassen eines Positiv-Kontrollsignals;
b2) Verändern der Stringenzbedingungen bei T=T[mess], so dass:

$$Tm(K) < T[mess] < Tm(Z),$$

und erfassen eines Messsignals; und optional
c) Verändern der Stringenzbedingungen, so dass T[n] > Tm(Z), und erfassen eines Negativ-Kontrollsignals.

Tm(K) ist die Schmelztemperatur der Kontrollnukleinsäure bei gegebenen Lösungsmittelbedingungen.
Tm(Z) ist die Schmelztemperatur der Zielnukleinsäure bei gegebenen Lösungsmittelbedingungen.
T[n] ist die Temperatur, bei der die Messung des Negativkontrollsignals durchgeführt wird.
T[p] ist die Temperatur, bei der die Messung des Positiv-Kontrollsignals durchgeführt wird.
T[mess] ist die Temperatur, bei der die Messung eines Messsignals durchgeführt wird.
Bevorzugt wird das Verändern der Stringenzbedingungen durch Erhöhen der Temperatur erreicht. Alternativ kann es durch Verändern der Lösungsmittelbedingungen oder durch Verändern einer Kombination der Lösungsmittelbedingungen und Temperatur erreicht werden.
Gemäß einem weiteren Aspekt der Erfindung können mehrere Messsignale 1 bis n bei unterschiedlichen Temperaturen T[mess 1-n] gemessen werden, wobei Tm(K) < T[mess 1-n] < Tm(Z).
Gemäß einem weiteren Aspekt der Erfindung kann das Verfahren für eine Mehrzahl von in einer Arrayanordnung angeordneten Sensorelementen durchgeführt werden.
Gemäß einem weiteren Aspekt der Erfindung können Zielnukleinsäure und Kontrollnukleinsäure mit einem detektierbaren Label markiert werden.
Zielnukleinsäure und Kontrollnukleinsäure können direkt markiert sein (z.B. mit einem Farbstoff, Enzym, o.ä.), sie können aber auch indirekt markiert sein (z.B. mit Biotin, einer Erkennungssequenz für einen sekundären Bindungspartner mit Label o.ä.).
Gemäß einem weiteren Aspekt der Erfindung kann das detektierbare Label ein enzymatisches Label sein, welches ein Substrat zu einem durch das Sensorelement detektierbaren Produkt umsetzen kann.
Gemäß einem weiteren Aspekt der Erfindung kann vor Schritt (a) das Sensorelement mit detektierbaren Produkt in Kontakt gebracht werden, um ein erstes Kalibrierungssignal zu erfassen.
Gemäß einem weiteren Aspekt der Erfindung kann auf einem weiteren Sensorelement, auf welchem detektierbares Label direkt immobilisiert ist, zusätzlich ein Temperatur-Kompensationssignal jeweils bei den Temperaturen T[p], T[mess], T[mess 1-n] und T[n] erfasst werden.
Gemäß einem weiteren Aspekt der Erfindung kann das Sensorelement ein elektrochemisches Sensorelement sein und Zielnukleinsäure und Kontrollnukleinsäure mit einem detektierbaren Label markiert werden, welches ein enzymatisches Label ist, welches ein Substrat zu einem durch das Sensorelement detektierbaren Produkt umsetzt.
Das enzymatische Label kann insbesondere eine Phosphatase sein.
Gemäß einem weiteren Aspekt der Erfindung ist die Schmelztemperatur Tm(K) der Kontrollnukleinsäure (K) bevorzugt um mindestens 5°C kleiner, stärker bevorzugt mindestens 10°C kleiner als die Schmelztemperatur Tm(Z) der Zielnukleinsäure (Z). Gemäß einem weiteren Aspekt der Erfindung kann bevorzugt als Kontrollnukleinsäure eine Nukleinsäurezusammensetzung verwendet werden, welche randomisierte Decaoligonukleotidsequenzen aufweist. Die Verwendung von randomisierten 6, 7, 8, 9, 11, 12, 13, 14, oder 15-meren ist auch möglich. Insbesondere sind biotinylierte

Oligomere bevorzugt. Ebenfalls möglich sind Kontrollnukleinsäuren, welche eine spezifische Erkennungssequenz tragen, die an eine spezifische komplementäre Sequenz an dem Sondenolignukleotids des Sensorelements bindet, wobei diese komplementäre Sequenz des Sondenolignukleotids nicht an die Zielnukleinsäure bindet.

Offenbart wird ferner eine Anordnung, aufweisend:

- mindestens ein Sensorelement mit einem darauf immobilisierten Sondennukleotid, über welches die Bindung einer Ziel-Nukleinsäure (Z) durch das Sensorelement erfasst werden kann, wobei ein Bindungskomplex aus Sondenoligonukleotid und Ziel-Nukleinsäure eine Schmelztemperatur Tm(Z) aufweist;
- Heiz- und /oder Kühlmittel zur Veränderung der Temperatur an dem Sensorelement;
- mindestens eine Kontrollnukleinsäure, welche mit dem Sondennukleotid ein Bindungskomplex bilden kann, der eine Schmelztemperatur von Tm(K) < Tm(Z) aufweist.

Gemäß einem weiteren Aspekt weist die Anordnung ferner eine Mehrzahl von in einer Arrayanordnung angeordneten Sensorelementen auf. Die Sensorelemente können als Microarray vorgesehen sein, z.B. als Biochip. Die Arrayanordnung kann als Teil einer mikrofluidischen Vorrichtung ausgeführt sein. Eine mikrofluidische Vorrichtung ist eine Vorrichtung, in welcher Fluide, insbesondere Flüssigkeiten in Volumina im Mikroliterbereich gehandhabt werden können.

Gemäß einem weiteren Aspekt ist die Kontrollnukleinsäure in der Anordnung als Trockenreagenz bevorratet. Durch Einbringen eines entsprechenden Lösungsmittels (z.B. eine Pufferlösung) wird die Kontrollnukleinsäure gelöst und kann dann an Sondenoligonukleotide binden.

Gemäß einem weiteren Aspekt ist die Anordnung in Form eines Kits vorgesehen, umfassend eine Vorrichtung, welche die Heiz- und oder Kühlmittel und das mindestens ein Sensorelement umfasst, und umfassend eine Zusammensetzung, welche die Kontrollnukleinsäure aufweist.

Gemäß einem weiteren Aspekt ist die Kontrollnukleinsäure mit einem detektierbaren Label markiert.

Gemäß einem weiteren Aspekt ist das detektierbare Label ein enzymatisches Label, welches ein Substrat zu einem durch das Sensorelement detektierbaren Produkt umsetzen kann.

Gemäß einem weiteren Aspekt ist auf einem weiteren Sensorelement detektierbares Label direkt immobilisiert.

Gemäß einem weiteren Aspekt ist das Sensorelement der Anordnung ein elektrochemisches Sensorelement und die Kontrollnukleinsäure ist mit einem detektierbaren Label markiert, welches ein enzymatisches Label ist, welches ein Substrat zu einem durch das Sensorelement detektierbaren Produkt umsetzt.

Gemäß einem weiteren Aspekt weist die Anordnung als Kontrollnukleinsäure eine Nukleinsäurezusammensetzung auf, welche randomisierte Decaoligonukleotidsequenzen aufweist.

Gemäß einem weiteren Aspekt sind die Nukleinsäuren mit randomisierten Decaoligonukleotidsequenzen biotinyliert.

Im Sinne der vorliegenden Erfindung ist ein Sensorelement ein Element, welches bei Bindung einer Ziel-Nukleinsäure (Z) an ein mit dem Sensorelement assoziiertes Sondenoligonukleotid ein messtechnisch verwertbares Signal erfassen kann. Dieses Signal kann ein qualitatives oder ein quantitatives Signal sein. Bevorzugt ist das Signal quantitativ, d.h. die Signalstärke korreliert mit der Menge der gebundenen Ziel-Nukleinsäuren.

Ein Sondenoligonukleotid ist ein Oligonukleotid, welches als Sonde zur Bindung einer Ziel-Nukleinsäure dient. Bevorzugt ist als Sondenoligonukleotid eine einzelsträngige Nukleinsäure. Das Sondennukleotid kann mit DNA, RNA oder synthetischen oder modifizierten Nukleotidanaloga aufgebaut sein. Bevorzugt hat das Sondenoligonukleotid eine definierte Sequenz. Bevorzugt hat das Sondenoligonukleotid eine Länge von mehr als 10 Nukleotiden, stärker bevorzugt mehr als 20 Nukleotiden. Ein "immobilisiertes" Sondenoligonukleotid bedeutet ein Nukleotid, welches auf dem Sensorelement oder in ausreichend räumlicher Nähe zu dem Sensorelement räumlich fixiert ist, derart, dass es unter den Bedingungen, unter welchen Messungen an diesem Sondensensorelement durchgeführt werden, seine Position an dem Sensorelement oder in ausreichender Nähe zu dem Sensorelement beibehält. Das Sondenoligonukleotid kann direkt an dem Sensorelement aufgebracht sein, z.B. auf der Elektrode eines elektrochemischen Sensorelements oder in unmittelbarer räumlicher Nähe zu dem Sensorelement, es kann indirekt, z.B. über ein Spacer-Molekül immobilisiert sein oder durch ein Kompartiment immobilisiert sein, d.h. durch eine Abgrenzung oder Umschließung. Eine Kontrollnukleinsäure im Sinne der vorliegenden Erfindung ist eine Nukleinsäure, welche ebenfalls an das Sondenoligonukleotid hybridisieren kann, wobei allerdings das Hybrid aus Sondenoligonukleotid und Kontrollnukleinsäure (K) eine geringere Schmelztemperatur als der Hybrid aus Sondenoligonukleotid und Ziel-Nukleinsäure (Z) aufweist. Dies wird üblicherweise dadurch erreicht, dass die Länge des komplementären Strangs, über welchen Köntrollnukleinsäure und Sondenoligonukleotid hybridisieren, kürzer ist als die Stranglänge, über welche die Ziel-Nukleinsäure mit dem Sondenoligonukleotid hybridisiert. Hierauf wird im Folgenden noch genauer eingegangen. Der Begriff "hybridisieren" umfasst das In-Kontakt-Bringen von zwei Nukleinsäuren (z.B. Sondenoligonukleotid und Kontroll-Nukleinsäure) unter Bedingungen (Temperatur, pH-Wert, Pufferzusammensetzung, Salzkonzentration), welche die Ausbildung von Hybriden über komplementäre Stränge ermöglichen.

Der Begriff "Erfassen eines Signals" umfasst alle Schritte und Voraussetzungen, welche notwendig sind, damit an dem Sensorelement ein Signal abgeleitet werden kann, welches auf die Bindung einer Nukleinsäure an das Sondenoligonukleotid anzeigen kann. Bei einer direkten Markierung, Markierung der Ziel-Nukleinsäure mit einem Farbstoff, kann das Signal z.B. direkt mit Hilfe einer CCD-Kamera erfasst werden. Bei einer indirekten Markierung, z.B. durch Biotinylierung der Ziel-Nukleinsäure oder Kontroll-Nukleinsäure, sollen im Sinne der vorliegenden Erfindung alle weiteren erforderlichen Schritte (Inkubation mit Streptavidin-Enzymkomplex, Waschschritte, Inkubation mit Enzymsubstrat und Messen des Messsignals) durch den Begriff "Erfassen eines Signals" mit umfasst sein.

Unter dem Begriff Positiv-Kontrollsignal wird im Sinne der vorliegenden Erfindung ein Signal verstanden, welches unter den jeweils gegebenen Bedingungen (Temperatur etc.) der maximalen Signalstärke entspricht, welche an dem Sensorelement abgeleitet werden kann. Dies ist z.B. dann der Fall, wenn sämtliche individuellen Sondenoligonukleotid-Moleküle einen Bindungspartner gebunden haben.

Mit dem Begriff Negativ-Kontrollsignal wird im Kontext der vorliegenden Erfindung ein Signal bezeichnet, welches für das jeweilige Sensorelement unter den jeweiligen Messbedingungen einem Minimalsignal oder einem Hintergrundrauschen entspricht. Dies ist z.B. dann der Fall, wenn keine der einzelnen Sondenoligonukleotid-Moleküle bei Erfassen des Signals einen Bindungspartner gebunden hat.

Eine Arrayanordnung bedeutet eine Anordnung von einzelnen Sensorelementen, welche in einzeln adressierbaren Positionen angeordnet sind.

Ein detektierbares Label bedeutet im Kontext der vorliegenden Erfindung eine Markierung, durch welche direkt oder indirekt ein messbares Signal erfasst werden kann. Dies kann ein direktes Label sein, z.B. ein Farbstoff, Fluoreszenzfarbstoff, radioaktive Markierung etc. Ebenso ist durch den Begriff detektierbares Label jedoch eine indirekte Markierung umfasst, bei welcher zur Erfassung eines messbaren Signals weitere Schritte erforderlich sind, z.B. Biotin-Markierung, Antikörper-Markierung, Enzym-Markierung etc.

Die Wahl einer Sequenz und deren Länge in Basenpaaren für ein Sondenolignukleotid hängt von einer Vielzahl an Überlegungen ab. Die Länge einer Sondenolignukleotidsequenz darf eine bestimmte Länge nicht überschreiten. Mit der Länge des Sondenolignukleotids wächst die Schmelztemperatur des Duplexes aus Sondenolignukleotid und Zielnukleinsäure. Die Schmelztemperatur (Tm) ist diejenige Temperatur, bei der das Sondenolignukleotid sich von der Zielnukleinsäure löst (schmilzt), oder anders ausgedrückt, unterhalb seiner Schmelztemperatur hybridisiert es mit der Zielnukleinsäure. Die Schmelztemperatur eines Nukleinsäuredoppelstrangs hängt bei konstanten äusseren Bedingungen (Lösungsmittelbedingungen) allerdings nicht nur von seiner Länge ab, sondern auch von seiner Nukleotidzusammensetzung. G-C-Basenpaarungen werden durch drei Wasserstoffbrücken stabilisiert, T-A-Basenpaarungen nur über zwei. G-C-Basenpaarungen sind also "stabiler". Die Schmelztemperatur nimmt also mit der Anzahl an G-Nukleotiden und C-Nukleotiden stärker zu. Die Schmelztemperatur lässt sich mit mehreren Methoden berechnen, die Angaben beziehen sich jeweils auf °C:

Die GC-Methode ist eine einfache aber auch ungenaue Methode:

$$Tm\ (^\circ C) = (64 + 41*(\%GC-16.4))^\circ C$$

wobei %GC der Anteil an G und C Basen an der Gesamtzahl der Basen ist.

Die "salt adjusted"-Methode ist etwas genauer und bezieht die Konzentration an Na+-Ionen im Reaktionsansatz mit ein:

$$Tm\ (^\circ C) = (100{,}5 + 41*\%GC - (820:L) * 16{,}6\ \log[Na+])^\circ C$$

Wobei L die Gesamtzahl der Basen ist und wobei [Na+] die Konzentration an Na+ Ionen angibt.

Eine weitere Methode ist die "base stacking" Methode, bei der die Enthalpie- und Entropieterme der Helixbildung bei der Hybridisierung mit einbezogen werden.

Auch weitere Bestandteile des Lösungsmittels haben einen Einfluss auf die Schmelztemperatur. Insbesondere, wenn Formamid verwendet wird, um die Hybridisierungsbedingungen zu steuern. Mit folgender Formel lässt sich die Temperatur unter Berücksichtigung dieser Parameter abschätzen:

$$Tm = 81{,}5 + 0{,}41 * (\%\ GC) + 16{,}6 \log [Na+] - (820:L) - 0{,}61 * (\%\ Formamid) - 1{,}4\ (\%\ mismatch)$$

Wobei % mismatch = Anteil der fehlgepaarten Basen. Die Hybridisierungstemperatur sollte optimalerweise etwa 5-10°C unter der Schmelztemperatur für DNA/DNA-Hybride sein, bei DNA/RNA-Hybriden reichen 5°C.

Für kürzere Sequenzen werden andere Näherungsformeln verwendet. Um die Hybridisierungs-Temperatur zu schätzen, hat sich in der Praxis die (4+2)-Regel bewährt: Dabei werden für jedes Cytosin und jedes Guanin 4°C gezählt, für jedes Adenin und jedes Thymidin 2°C. Diese Summe abzüglich 5-10°C ergibt die ungefähre Hybridisierungs-Temperatur. Die verschiedenen Formeln zur näherungsweisen Berechnung der Schmelztemperatur zeigen, dass neben der Sondenlänge (Länge des komplementären Bereichs) auch GC Gehalt, Salzkonzentration und Lösungsmittelbeschaffenheit die Schmelztemperatur bestimmen. Die Kombination dieser Parameter stellt die Stringenzbedingungen dar, welche darüber entscheiden, ob zwei komplementäre oder zumindest abschnittsweise komplementäre Nukleinsäurestränge hybridisieren oder nicht. Entsprechend können durch Variation der Parameter Temperatur, Salzkonzentration, Lösungsmittelzusammensetzung die Stringenzbedingungen verändert werden und die Hybridisierung (auch "Annealing" bezeichnet) bzw. das Schmelzen oder Auflösen der Doppelstränge gesteuert werden.

Mittlerweile gibt es auch eine große Anzahl an Software, mit der man die Schmelztemperatur von Sequenzen berechnen kann.

Die Erfindung wird nun anhand von Beispielen und den angehängten Figuren veranschaulichend und beispielhaft beschrieben. In den Figuren zeigen:

Figur 1: eine schematische Darstellung eines beispielhaften Sensorelements, wie es bei dem erfindungsgemäßen Verfahren Verwendung findet;

Figur 2: eine schematische Darstellung einer ersten Ausführungsform der Erfindung;

Figur 3: eine schematische Darstellung einer weiteren Ausführungsform der Erfindung;

Figur 4: eine schematische Darstellung einer weiteren Ausführungsform der Erfindung; und

Figur 5: eine schematische Darstellung einer weiteren Ausführungsform der Erfindung.

Figur 6: eine schematische Darstellung einer weiteren Ausführungsform der Erfindung.

In der Figur 1 ist ein beispielhaftes Sensorelement mit einer Messanordnung mit zwei Elektroden 2 und 3, wobei zusätzlich eine Goldfläche 5 vorhanden ist, auf welcher ein Sondenoligonukleotid 100 immobilisiert ist. Alternativ können entsprechende Sondenoligonukleotide auch direkt auf den Elektroden 2,3 immobilisiert sein. Entsprechende Sensorelemente sind beispielsweise ausführlich in DE 101 26 341 A1 beschrieben.

Figur 1 zeigt schematisch ein Substrat 1 mit planarer Oberfläche, welches beispielsweise durch die kristallographische Oberfläche eines Silizium-Chips gebildet wird. Auf dem Substrat 1 ist ein Array von elektrochemischen Detektoren 2, 3, auf vorgegebenen Arraypositionen realisiert, mit denen bioanalytische Untersuchungen mit enzymgekoppelten Reaktionen vorgenommen werden. Im Einzelnen ist für die bioanalytischen Untersuchungen ein Sondenoligonukleotid mit 100, ein AnalytMolekül mit 200 und ein sog. Enzym-Label mit 300 bezeichnet. Dabei reagiert das Sondenoligonukleotid 100 spezifisch mit einem komplementären Analytmolekül 200 (dies kann eine Zielnukleinsäure oder Kontrollnukleinsäure sein) und immobilisiert so arraypositionsspezifisch einen Enzym-Label 300. Anschließend zugegebenes Enzym-Substrat 400 wird durch die katalytische Wirkung des Enzym-Labels in ein Produkt 500 überführt.

Auf dem Sensorelement kann mit Hilfe der Elektroden 2, 3 die Abnahme/Zunahme von Substrat/Produkt gemessen werden. Als detekierbares Produkt 500 sei als Beispiel das Redoxpaar p-Aminophenol/Chinonimin genannt:

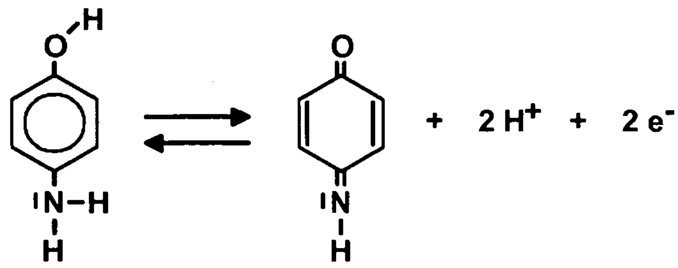

p-Aminophenol Chinonimin

Am entsprechenden Redoxprozess sind 2 Elektronen sowie 2 $H^+$-Ionen beteiligt.

Dieses System kommt z.B. bei Enzym-gekoppelten Nachweisreaktionen zum Einsatz. Dabei wird das Enzym "Alkalische Phosphatase" als Label- bzw. Verstärkungs-Substanz eingesetzt. Alkalische Phosphatase ist in der Lage, p-Aminophenyl-Phosphat in p-Aminophenol und Phosphat zu spalten:

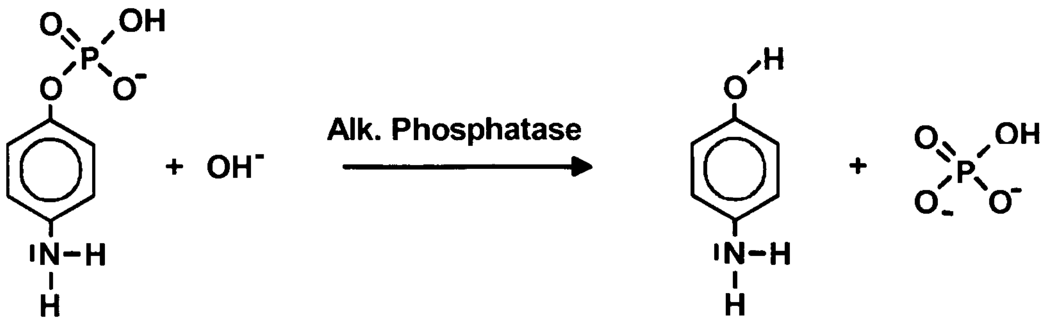


p-Aminophenyl-Phosphat

Das entstehende p-Aminophenol wird am Elektroden-System oxidiert bzw. das Redoxpaar p-Aminophenol/ Chinonimin zyklisiert. Das Signal kann dann als Anstieg der Stromstärke (I) im Verlauf der Zeit (t) an den Elektroden abgeleitet werden, wobei die Signalstärke S =dI/dt.

In Figur 2 ist eine schematische Darstellung einer ersten Ausführungsform der Erfindung gezeigt. Ein Sensorelement wird mit Kontrollnukleinsäuren (K) beladen bei einer Temperatur T[p]<Tm(K), wobei Tm(K) die Schmelztemperatur des Duplexes aus Sondenoligonukleotid und Kontrollnukleinsäure unter gegebenen Lösungsmittelbedingungen ist. Die Konzentration der Kontrollnukleinsäure ist ausreichend hoch, dass alle freien Sondenolignukleotide abgesättigt werden können. Bei der Temperatur T[p] hybridisieren die Kontrollnukleinsäure-Moleküle an sämtliche freie Sondenolignukleotide. Die Kontrollnukleinsäure ist mit Biotin (B) gelabelt bzw. markiert. Nun wird Streptavidin-konjugiertes Enzym (E) zugegeben, welches an Biotin (B) bindet und ein Substrat (nicht gezeigt) umsetzen kann, so dass an dem Sensorelement ein Signal (+) abgeleitet werden kann.

Bei der Temperatur T[p] sind alle Sondenolignukleotide mit Kontrollnukleinsäure abgesättigt, so dass man ein maximales Signal als Positiv-Kontrollsignal erhält.

Anschließend wird die Temperatur auf T[n]>Tm(K) erhöht. Die Duplexe aus Sondenoligonukleotid und Kontrollnukleinsäure schmelzen auf, so dass die Kontrollnukleinsäuren freigesetzt werden und (zusammen mit dem Enzym weggespült werden können. Bei der Temperatur T[n] sind alle Sondenolignukleotide nun wieder ungesättigt, so dass kein Signal (-) bzw. lediglich ein Rauschsignal gemessen wird und man ein minimales Signal als Negativ-Kontrollsignal erhält.

Die Werte für das Positiv-Kontrollsignal und das Negativ-Kontrollsignal können z.B. in einer Steuereinrichtung gespeichert werden und als zukünftige Referenzwerte weiter benutzt werden. Man kann auf diese Weise eine spezifische Kalibrierung für die einzelnen Positionen eines Microarray durchführen, bei dem jede Arrayposition (jeder "Spot") durch das in Fig. 2 dargestellte Verfahren kalibriert wird. Dies kann auch zur Qualitätskontrolle des Microarrays durchgeführt werden.

In Figur 3 ist eine schematische Darstellung einer weiteren Ausführungsform der Erfindung gezeigt. Ein Sensorelement wird mit einer Probe beladen, welche mutmaßlich die Zielnukleinsäure (Z) für ein entsprechendes Sondenoligonukleotid enthält. Ferner wird Kontrollnukleinsäure (K) mit hinzu gegeben. Die Temperatur beträgt T[p]<Tm(K), wobei Tm(K) die Schmelztemperatur des Duplexes aus Sondenoligonukleotid und Kontrollnukleinsäure unter gegebenen

Lösungsmittelbedingungen ist (zusätzlich gilt Tm(K)< Tm[Z]). Die Konzentration der Mischung aus Zielnukleinsäure und Kontrollnukleinsäure ist ausreichend hoch, dass alle freien Sondenolignukleotide mit Zielnukleinsäure oder Kontrollnukleinsäure abgesättigt werden können. Bei der Temperatur T[p] hybridisieren die Ziel- und Kontrollnukleinsäure-Moleküle an sämtliche freie Sondenolignukleotide. Die Kontrollnukleinsäure ist mit Biotin (B) gelabelt. Nun wird Streptavidin-konjugiertes Enzym (E) zugegeben, welches an Biotin (B) bindet und ein Substrat (nicht gezeigt) umsetzen kann, so dass an dem Sensorelement ein Signal (+) abgeleitet werden kann.

Bei der Temperatur T[p] sind nun alle Sondenolignukleotide mit Ziel- oder Kontrollnukleinsäure abgesättigt, so dass man ein maximales Signal als Positiv-Kontrollsignal erhält.

Anschließend wird die Temperatur T[mess]auf Tm(K)<T[mess]<Tm(Z) erhöht, wobei Tm(Z) die Schmelztemperatur des Duplexes aus Sondenoligonukleotid und Kontrollnukleinsäure unter gegebenen Lösungsmittelbedingungen ist. Die Duplexe aus Sondenoligonukleotid und Kontrollnukleinsäure schmelzen auf, so dass die Kontrollnukleinsäuren freigesetzt werden und (zusammen mit dem daran gebundenen Enzym) weggespült werden können. Bei der Temperatur T[mess] liefern nur noch die Sondenoligonukleotide, die mit Zielnukleinsäure belegt sind, ein Signal (+), welches ein Messsignal für die Zielnukleinsäure ist.

Anschließend wird nun die Temperatur auf T[n]>Tm(Z) erhöht. Die Duplexe bzw. Hybride aus Sondenoligonukleotid und Zielnukleinsäure schmelzen jetzt auch auf, so dass die Zielnukleinsäuren freigesetzt werden und (zusammen mit dem Enzym) weggespült werden können. Bei der Temperatur T[n] sind alle Sondenoligonukleotide nun wieder ungesättigt, so dass kein Signal (-) bzw. lediglich ein Rauschsignal gemessen wird und man ein minimales Signal als Negativ-Kontrollsignal erhält.

Der Signalstärkewert der Zielnukleinsäure S[Z] kann beispielsweise dann in Relation zu dem Positiv-Kontrollsignal und Negativ-Kontrollsignal ausgedrückt werden, z.B. gemäß der Formel:

$$S[Z]=(S[mess]-S[n])/(S[p]-S[n]).$$

Auf diese Weise kann bei jeder Messung eine gleichzeitige Kalibrierung des Sensorelements durchgeführt werden, was eine besonders hohe Messgenauigkeit ermöglicht.

Die Sequenz der Kontrollnukleinsäure ist z.B. so gewählt, dass sie über eine kürzere komplementäre Sequenz an das Sondenoligonukleotid bindet, als die Zielnukleinsäure. Alternativ kann die Kontrollnukleinsäure auch einen geringeren GC-Gehalt oder eine Kombination aus geringerem GC-Gehalt und kürzerer komplementär bindender Sequenz als die Zielnukleinsäure aufweisen. In den Figuren 2 bis 6 ist dies lediglich schematisch dargestellt. Lediglich aus Gründen der Übersichtlichkeit ist die Zielnukleinsäure mit fünf bindenden Basenpaaren und die Kontrollnukleinsäure mit zwei bindenden Basenpaaren dargestellt. Bevorzugt bindet die Zielnukleinsäure über einen Bereich von 15 oder mehr, stärker bevorzugt 30 oder mehr bindenden Basenpaaren.

Wenn die Zielnukleinsäure über einen komplementären Bereich von ca. 20 bis 25 Basen an das Sondenoligonukleotid bindet, so kann für die Kontrollnukleinsäure der komplementäre Bereich beispielsweise mit einer Länge von 6 bis 12 Basen gewählt werden. Es ist möglich, eine Zielnukleinsäure zu wählen, welche im komplementären Bereich teilweise eine zur Zielnukleinsäure identische Sequenz aufweist, so dass sie über teilweise dieselben Nukleotide im Sondenoligonukleotid wie die Zielnukleinsäure bindet. Es ist auch denkbar, als Kontrollnukleinsäure kurze Oligomere mit randomisierten Sequenzen zu verwenden, z.B. randomisierte Hexamere, randomisierte Decamere oder ähnliche.

Gemäß einer alternativen Ausführungsform weist das Sondenoligonukleotid einen Bereich mit einer Sequenz auf, die nicht an die Zielnukleinsäure hybridisieren kann, sondern nur an die Kontrollnukleinsäure. Gemäß einer weiteren Ausführungsform kann dieser Bereich durch einen Spacer von der Sequenz, welche die Zielnukleinsäure bindet, beabstandet sein, so dass es sogar möglich ist, dass Zielnukleinsäure und Kontrollnukleinsäure gleichzeitig an das gleiche Sondenoligonukleotidmolekül binden. Dies ist z.B. in Figur 4 dargestellt, wo an ein Sondenoligonukleotid sowohl die kürzere Kontrollnukleinsäure als auch die längere Zielnukleinsäure an unterschiedliche Bereiche des Sondenolignukleotids binden können. Das Verfahren gemäß Fig. 4 wird ansonsten entsprechend dem Verfahren gemäß Fig. 3 durchgeführt.

In Fig. 5 ist eine schematische Darstellung einer weiteren Ausführungsform der Erfindung gezeigt, bei dem eine Schmelzpunktkurve der Zielnukleinsäure (Z) bestimmt wird. Ein Sensorelement wird mit einer Probe beladen, welche mutmaßlich die Zielnukleinsäure für ein entsprechendes Sondenoligonukleotid enthält. Ferner wird - wie beim Verfahren gemäß Fig. 3 Kontrollnukleinsäure (K) mit hinzugegeben. Die Temperatur beträgt T[p]<Tm(K), wobei Tm(K) die Schmelztemperatur des Duplexes aus Sondenoligonukleotid und Kontrollnukleinsäure unter gegebenen Lösungsmittelbedingungen ist. Die Konzentration der Mischung aus Zielnukleinsäure und Kontrollnukleinsäure ist ausreichend hoch, so dass alle freien Sondenolignukleotide mit Zielnukleinsäure oder Kontrollnukleinsäure abgesättigt werden können. Bei der Temperatur T[p] hybridisieren die Ziel- und Kontrollnukleinsäure-Moleküle an sämtliche freie Sondenolignukleotide. Die Kontrollnukleinsäure ist mit Biotin (B) gelabelt. Nun wird Streptavidin-konjugiertes Enzym (E) zugegeben, welches an Biotin (B) bindet und ein Substrat (nicht gezeigt) umsetzen kann, so dass an dem Sensorelement ein Signal (+) abgeleitet werden kann.

Bei der Temperatur T[p] sind nun alle Sondenolignukleotide mit Ziel- oder Kontrollnukleinsäure abgesättigt, so dass man ein maximales Signal als Positiv-Kontrollsignal erhält.

Anschließend wird die Temperatur schrittweise über T[mess 1] T[mess 2] etc. erhöht auf T[p]<T[mess 1-n]<Tm (Z) und wobei Tm(Z) die Schmelztemperatur des Duplexes aus Sondenoligonukleotid und Zielnukleinsäure unter gegebenen Lösungsmittelbedingungen ist. Die Duplexe aus Sondenoligonukleotid und Kontrollnukleinsäure schmelzen als erstes (wie in Fig. 5 bei T[mess 1] dargestellt) auf, so dass die Kontrollnukleinsäuren freigesetzt werden und (zusammen mit dem daran gebundenen Enzym) weggespült werden können. Bei der Temperatur T[mess 1] liefern nur noch die Sondenoligonukleotide, die mit Zielnukleinsäure belegt sind, ein Signal, welches ein Messsignal für die Zielnukleinsäure ist. Bei der schrittweisen Temperaturerhöhung beginnen nun auch die Duplexe aus Sondenoligonukleotid und Zielnukleinsäure nach und nach aufzuschmelzen (wie in Fig. 5 bei T[mess 2] dargestellt), so dass eine Schmelzkurve erstellt werden kann.

Schließlich wird nun die Temperatur auf T[n]>Tm(Z) erhöht. Die Duplexe aus Sondenoligonukleotid und Zielnukleinsäure schmelzen jetzt vollständig auf, so dass sämtliche Zielnukleinsäuren freigesetzt werden und (zusammen mit dem Enzym) weggespült werden können. Bei der Temperatur T[n] sind alle Sondenoligonukleotide nun wieder ungesättigt, so dass kein Signal (-) bzw. lediglich ein Rauschsignal gemessen wird und man ein minimales Signal als Negativ-Kontrollsignal erhält.

Ferner kann mit Para-aminophenol (pAP), dem primären Substrat der elektrochemischen Reaktion, eine initiale Kalibrierungsmessung durchgeführt werden, um festzustellen, ob alle Elektroden auf das Vorhandensein dieser redoxreaktiven Substanz ansprechen. Damit kann verifiziert werden, dass sämtliche Elektroden auf dieses redoxreaktive Produkt ansprechen. Nun wird freies Para-aminophenol weggespült und mit einer Temperatur-abhängigen Messsequenz begonnen.

In Figur 6 ist eine schematische Darstellung einer weiteren Ausführungsform der Erfindung gezeigt. In der oberen Reihe ist die normale Messsequenz, so wie für Fig 5 beschrieben, zusammengefasst:

> Hybridisierung mit Zielnukleinsäure (Z) und Kontrollnukleinsäure (K), Messung der Positivkontrolle bei T[p]<Tm(K), Abschmelzen der Kontrollnukleinsäure und Erstellen der Schmelzkurve der Zielnukleinsäure bei T[p]<T[mess 1-n] <Tm(Z)und Erfassen des Signals der Negativkontrolle bei T[n]>Tm(Z). Auf weiteren Sensorelementen des Sensors, die als gesondert ausgelegte Spots zur Temperaturkontrolle ausgeführt sind, sind Sondenoligonukleotide aufgebracht, welche ein Biotin tragen (in Fig. 6 in der unteren Reihe dargestellt). Hier bindet das Enzym über den Komplex Biotin - Streptavidin über den gesamten gemessenen Temperaturbereich von T[p]<Tm(K), T[p]<T[mess 1-n]<Tm (Z), T[n]>Tm(Z) unabhängig von der Schmelztemperatur der DNA-Hybride an die Sondenoligonukleotide und liefert eine Aussage über die Abhängigkeit des Detektionssystems (Enzymaktivität und elektrochemische Redoxreaktion) von der Temperatur. Anhand des Temperaturverlaufs des Maximalsignales kann die Signalstärke auf den Mess-Spots, auf welchen die Zielnukleinsäure gemessen wird, korrigiert werden.

Ferner ist es möglich, zusätzliche Negativkontroll-Spots vorzusehen, welche mit Sondenoligonukleotiden mit randomisierten Sequenzen beschichtet sind, die also nicht spezifisch die Nukleinsäure binden können. Dadurch können unter anderem unspezifische Bindungseffekte und strömungsbedingte Temperatureffekte kompensiert werden. Schließlich wird jedes Sensor-Paar auf Enzym-Umsatz (Substrat-Turnover) in Mol/(Liter x sec.) kalibriert. Auch hier ist es wiederum möglich, aus der Signalstärke für den Mess-Spot (welcher Sondenoligonukleotide trägt, die für die Zielnukleinsäure spezifisch sind) und dem Negativkontroll-Spot ein kompensiertes Signal zu errechnen, z.B. durch Bildung von Differenz oder Quotient.

Es wird darauf hingewiesen, dass die Beispiele lediglich veranschaulichend und beispielhaft sind und dass im Rahmen des Schutzumfangs der Patentansprüche Änderungen und Modifikationen möglich sind. Insbesondere kann das erfindungsgemäße Verfahren in Verbindung mit anderen Sensorelementen und Markern oder Labeln verwendet werden, z.B. für optische, magnetische oder gravimetrische Sensorelemente.

## Patentansprüche

1. Verfahren zur Kalibrierung eines Sensorelements, welches ein immobilisiertes Sondenoligonukleotid aufweist, über welches die Bindung einer Ziel-Nukleinsäure (Z) durch den Sensor erfasst werden kann, aufweisend:

   a) In Kontakt Bringen des Sensorelements mit einer Mischung aufweisend Kontrollnukleinsäure (K) und Ziel-Nukleinsäure (Z), wobei die Schmelztemperatur der Kontrollnukleinsäure Tm(K) kleiner ist als die Schmelztemperatur der Zielnukleinsäure Tm(Z), wobei Tm (K) die Temperatur ist, unterhalb derer die Kontrollnukleinsäure (K) mit dem immobilisierten Sondenoligonukleotid hybridisiert und wobei Tm (Z) die Temperatur ist, unterhalb derer die Zielnukleinsäure (K) mit dem immobilisierten Sondenoligonukleotid hybridisiert;
b1) Hybridisieren der Mischung an das Sondenoligonukleotid bei einer Temperatur T[p] < Tm(K), und erfassen eines Positiv-Kontrollsignals;
b2) Verändern der Stringenzbedingungen bei einer Messtemperatur T=T[mess], so dass:

$$Tm(K) < T[mess] < Tm(Z),$$

und erfassen eines Messsignals; und
c) Verändern der Stringenzbedingungen, so dass T[n] > Tm(Z), und erfassen eines Negativ-Kontrollsignals.

**2.** Verfahren nach Anspruch 1, wobei das Verändern der Stringenzbedingungen durch Erhöhen der Temperatur erreicht wird.

**3.** Verfahren nach Anspruch 1, wobei mehrere Messsignale 1 bis n bei unterschiedlichen Temperaturen T[mess 1-n] gemessen werden, wobei Tm(K) < T[mess 1-n] < Tm(Z).

**4.** Verfahren nach einem der vorangehenden Ansprüche, wobei das Verfahren für eine Mehrzahl von in einer Arrayanordnung angeordneten Sensorelementen durchgeführt wird.

**5.** Verfahren nach einem der vorangehenden Ansprüche, wobei Zielnukleinsäure und Kontrollnukleinsäure mit einem detektierbaren Label markiert werden.

**6.** Verfahren nach Anspruch 5, wobei das detektierbare Label ein enzymatisches Label ist, welches ein Substrat zu einem durch das Sensorelement detektierbaren Produkt umsetzen kann.

**7.** Verfahren nach einem der vorangehenden Ansprüche, wobei vor Schritt (a) das Sensorelement mit detektierbaren Produkt in Kontakt gebracht wird, um ein erstes Kalibrierungssignal zu erfassen.

**8.** Verfahren nach einem der vorangehenden Ansprüche, wobei auf einem weiteren Sensorelement, auf welchem detektierbares Label direkt immobilisiert ist, zusätzlich ein Temperatur-Kompensationssignal jeweils bei den Temperaturen T[p], T[mess], T[mess 1-n] und T[n] erfasst wird.

**9.** Verfahren nach einem der vorangehenden Ansprüche, wobei das Sensorelement ein elektrochemisches Sensorelement ist und wobei Zielnukleinsäure und Kontrollnukleinsäure mit einem detektierbaren Label markiert sind, welches ein enzymatisches Label ist, welches ein Substrat zu einem durch das Sensorelement detektierbaren Produkt umsetzt.

**10.** Verfahren nach einem der vorangehenden Ansprüche, wobei die Schmelztemperatur Tm(K) der Kontrollnukleinsäure (K) um mindestens 5°C kleiner ist als die Schmelztemperatur Tm(Z)der Zielnukleinsäure (Z).

## Claims

**1.** Method for calibrating a sensor element having an immobilized probe oligonucleotide, via which the sensor can record binding of a target nucleic acid (Z), comprising:

a) contacting the sensor element with a mixture having control nucleic acid (K) and target nucleic acid (Z), with the melting temperature of the control nucleic acid, Tm(K), being lower than the melting temperature of the target nucleic acid, Tm(Z), Tm (K) being the temperature, below which the control nucleic acid (K) hybridizes with the immobilized probe oligonucleotide, and Tm (Z) being the temperature, below which the target nucleic acid (Z) hybridizes with the immobilized probe oligonucleotide;
b1) hybridizing the mixture to the probe oligonucleotide at a temperature T[p] < Tm(K), and recording a positive control signal;
b2) modifying the stringency conditions at a measuring temperature T=T[mess] such that:

$$Tm(K) < T[mess] < Tm(Z),$$

and recording a measurement signal; and
c) modifying the stringency conditions such that T[n] > Tm(K), and recording a negative control signal.

**2.** Method according to Claim 1, wherein the stringency conditions are modified by increasing the temperature.

**3.** Method according to Claim 1, wherein a plurality of measurement signals, 1 to n, are measured at different temperatures T[mess 1-n], where Tm(K) < T[mess 1-n] < Tm(Z).

**4.** Method according to any of the preceding claims, wherein the method is carried out for a majority of sensor elements arranged in an array arrangement.

**5.** Method according to any of the preceding claims, wherein target nucleic acid and control nucleic acid are labeled with a detectable label.

**6.** Method according to Claim 5, wherein the detectable label is an enzymatic label capable of converting a substrate into a product detectable by the sensor element.

**7.** Method according to any of the preceding claims, wherein step (a) is preceded by contacting the sensor element with detectable product in order to record a first calibration signal.

**8.** Method according to any of the preceding claims, wherein additionally a temperature compensation signal is recorded at each of the temperatures T[p], T[mess], T[mess 1-n] and T[n] on a further sensor element on which detectable label is immobilized directly.

**9.** Method according to any of the preceding claims, wherein the sensor element is an electrochemical sensor element, and wherein target nucleic acid and control nucleic acid are labeled with a detectable label which is an enzymatic label converting a substrate into a product detectable by the sensor element.

**10.** Method according to any of the preceding claims, wherein the melting temperature Tm(K) of the control nucleic acid (K) is at least 5°C lower than the melting temperature Tm(Z) of the target nucleic acid (Z).

## Revendications

**1.** Procédé d'étalonnage d'un élément capteur, qui comporte un oligonucléotide sonde immobilisé, par lequel la liaison d'un acide nucléique cible (Z) peut être détectée par le capteur, dans lequel :

a) on met l'élément capteur en contact avec un mélange comprenant de l'acide nucléique de contrôle (K) et de l'acide nucléique cible (Z), la température Tm(K) de fusion de l'acide nucléique de contrôle étant plus basse que la température Tm(Z) de fusion de l'acide nucléique cible, Tm(K) étant la température en dessous de laquelle l'acide (K) nucléique de contrôle s'hybride avec l'oligonucléotide sonde immobilisé et Tm(Z) étant la température en dessous de laquelle l'acide (K) nucléique cible s'hybride avec l'oligonucléotide sonde immobilisé ;
b1) on hybride le mélange sur l'oligonucléotide sonde à une température T[p] < Tm(K) et on détecte un signal de contrôle positif ;
b2) on modifie les conditions de stringence pour une température de mesure T=T[mess], telle que :

$$Tm(K) < T[mess] < Tm(Z),$$

et on détecte un signal de mesure ; et
c) on modifie les conditions de stringence de sorte que T[n] > Tm(Z),

et on détecte un signal de contrôle négatif.

**2.** Procédé suivant la revendication 1, dans lequel on obtient la modification des conditions de stringence par une élévation de la température.

**3.** Procédé suivant la revendication 1, dans lequel on mesure plusieurs signaux 1 à m de mesure à des températures T[mess 1-n] différentes, Tm(K) < T[mess 1-n] < Tm(Z).

**4.** Procédé suivant l'une des revendications précédentes, dans lequel on effectue le procédé pour une multiplicité d'éléments capteurs disposés suivant un agencement en matrice.

**5.** Procédé suivant l'une des revendications précédentes, dans lequel on marque de l'acide nucléique cible et de l'acide nucléique de contrôle par un marqueur détectable.

**6.** Procédé suivant la revendication 5, dans lequel le marqueur détectable est un marqueur enzymatique, qui peut transformer un substrat en un produit détectable par l'élément capteur.

**7.** Procédé suivant l'une des revendications précédentes, dans lequel, avant le stade (a), on met l'élément capteur en contact avec le produit détectable pour détecter un premier signal d'étalonnage.

**8.** Procédé suivant l'une des revendications précédentes, dans lequel, sur un autre élément capteur sur lequel un marqueur détectable est immobilisé directement, on détecte supplémentairement un signal de compensation de température, respectivement aux températures T[p], T[mess], T[mess 1-n] et T[n].

**9.** Procédé suivant l'une des revendications précédentes, dans lequel l'élément capteur est un élément capteur électrochimique et dans lequel de l'acide nucléique cible et de l'acide nucléique de contrôle sont marqués par un marqueur détectable, qui est un marqueur enzymatique qui transforme un substrat en un produit détectable par l'élément capteur.

**10.** Procédé suivant l'une des revendications précédentes, dans lequel la température Tm(K) de fusion de l'acide nucléique de contrôle (K) est plus basse d'au moins 5°C que la température de fusion Tm(Z) de l'acide nucléique (Z).

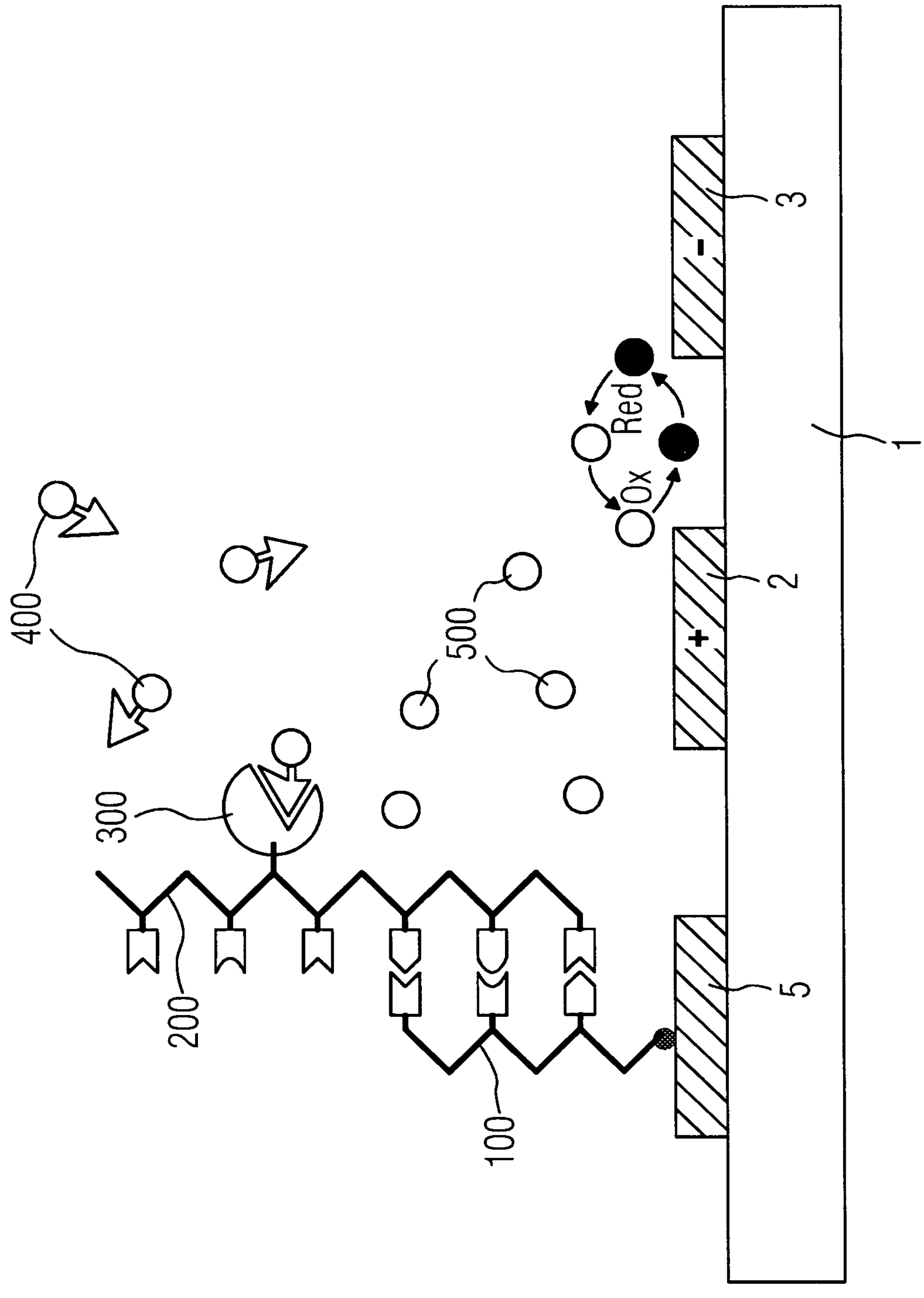


FIG 1

FIG 2

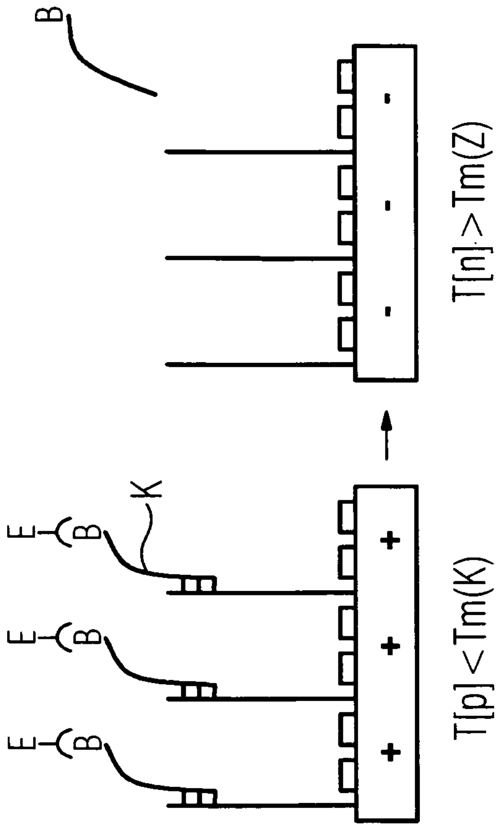


FIG 3

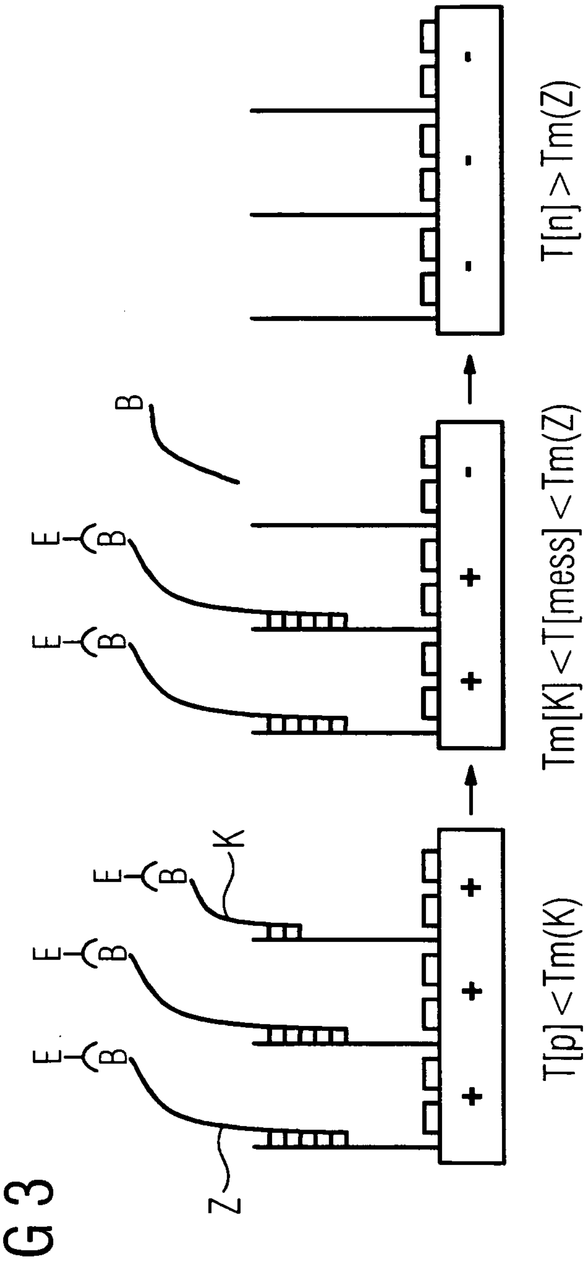

FIG 4

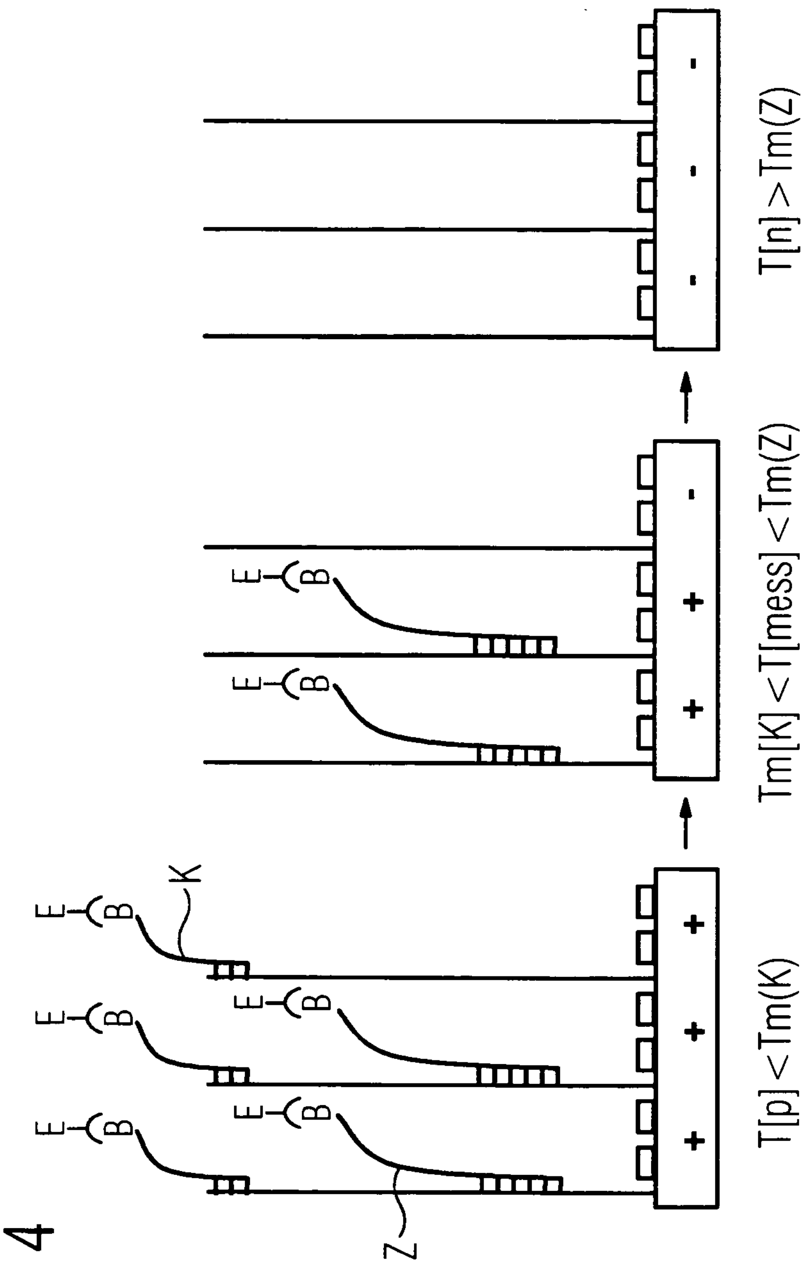

FIG 5

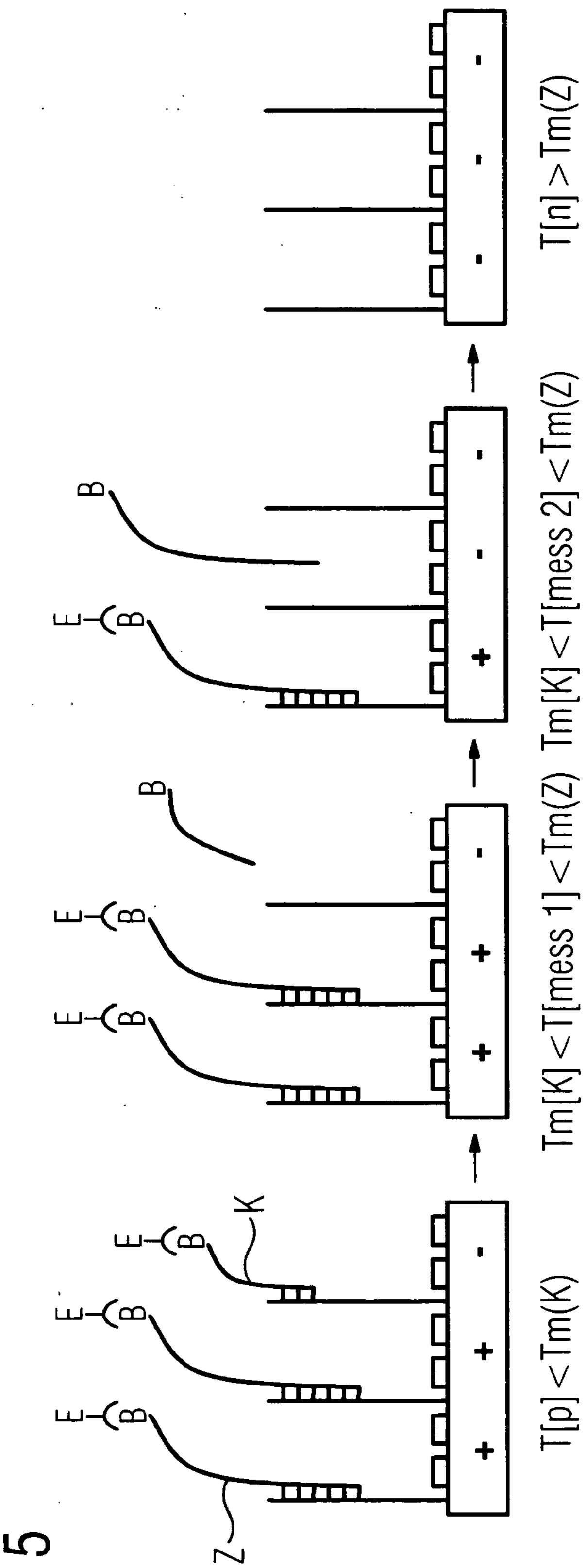

FIG 6

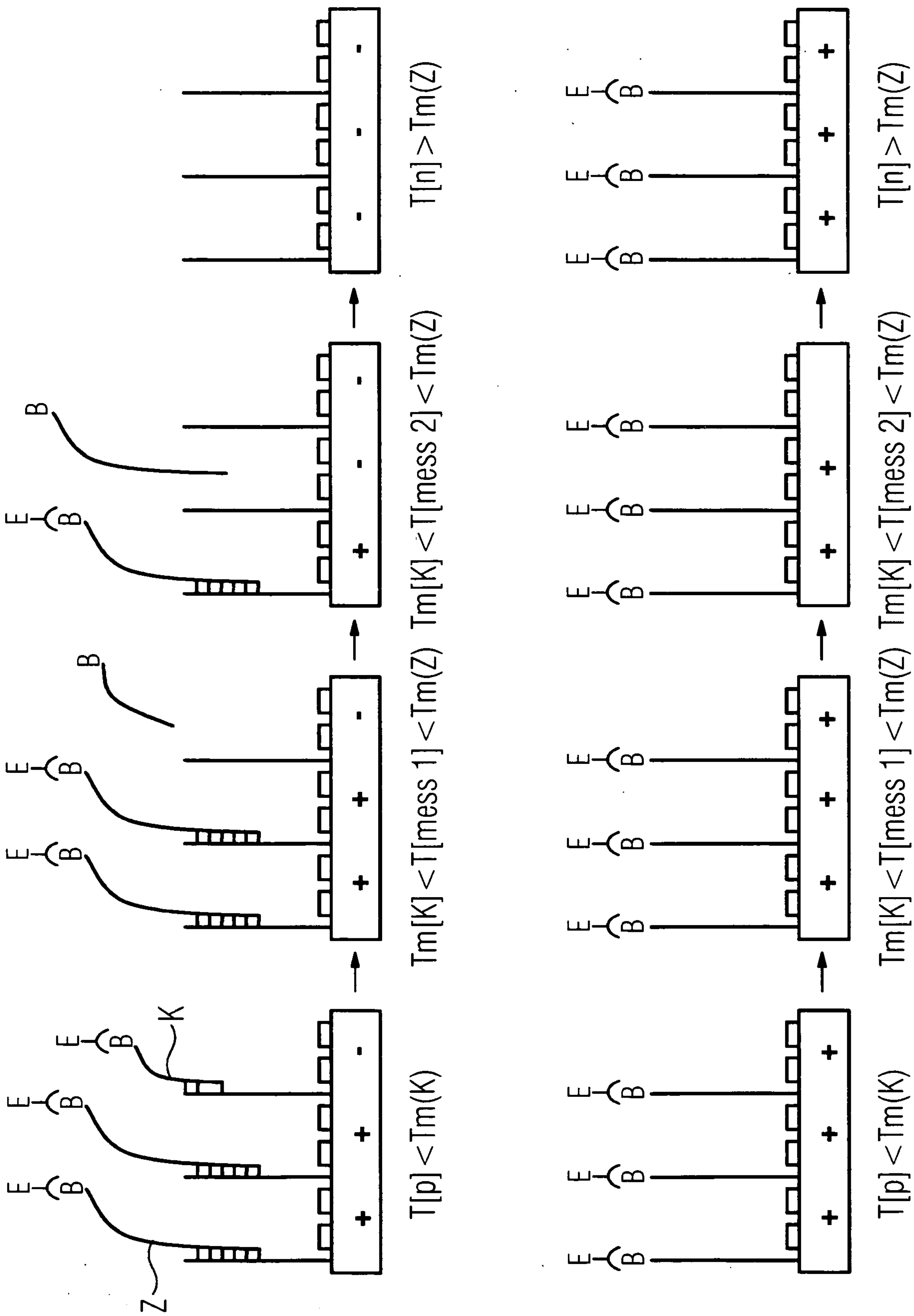
T[p]<Tm(K)
Tm[K]<T[mess 1]<Tm(Z)
Tm[K]<T[mess 2]<Tm(Z)
T[n]>Tm(Z)
T[p]<Tm(K)
Tm[K]<T[mess 1]<Tm(Z)
Tm[K]<T[mess 2]<Tm(Z)
T[n]>Tm(Z)
Z
K
E
B

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10126341
- WO 2005064012 A
- US 2006115851 A1
- DE 10126341 A1